# EUROPEAN PATENT SPECIFICATION

(11) **EP 1 238 676 B1**
(45) Date of publication and mention of the grant of the patent: **19.05.2004**
(21) Application number: 02251153.9
(22) Date of filing: 20.02.2002
(51) Int. Cl.: A61K 45/06, A61K 31/454, A61K 31/496, A61K 31/517, A61K 31/554, A61K 31/55, A61K 31/5513, A61P 25/18, A61P 25/22, A61P 25/24

(54) **Combination of a serotonin reuptake inhibitor and an atypical antipsychotic for use in depression, obsessive compulsive disorder and psychosis**
Kombination eines Inhibitoren der Serotoninwiederaufnahme und eines atypischen Antipsychotikums zur Behandlung von Depression, Zwangsneurosen und Psychosen
Combinaison d'un inhibiteur de la recapture de la sérotonine et d'un antipsychotique atypique pour l'utilisation dans la dépression, les troubles obsessionnels compulsifs et les psychoses

(30) Priority: 01.03.2001 US 272619 P
(43) Date of publication of application: 11.09.2002
(73) Proprietor: Pfizer Products Inc., Groton, Connecticut 06340 (US)
(72) Inventor: Howard, Harry Ralph, Pfizer Global Research & Dev., Groton, Connecticut 06340 (US)
(74) Representative: Wood, David John

(56) References cited:
- EP-A- 0 830 864
- WO-A-00/24399
- WO-A-00/50380

## Description

### Background Of The Invention

The present invention relates to a method of treating depression, obsessive compulsive disorder (OCD) and psychosis with improved efficacy in a mammal, including a human, by administering to the mammal an atypical antipsychotic agent in combination with a serotonin reuptake inhibitor (SRI). Selected from the compounds of the formula II as defined in the appended claims. It also relates to pharmaceutical compositions containing a pharmaceutically acceptable carrier, an atypical antipsychotic agent and a serotonin reuptake inhibitor (SRI). Selected from the compounds of the formula II as defined in the appended claims.

Major depression is characterized by feelings of intense sadness and despair, mental slowing and loss of concentration, pessimistic worry, agitation, and self-deprecation. Physical changes also occur, especially in severe or "melancholic" depression. These include insomnia or hypersomnia, anorexia and weight loss (or sometimes overeating), decreased energy and libido, and disruption of normal circadian rhythms of activity, body temperature, and many endocrine functions.

Serotonin Selective Reuptake Inhibitors (SSRIs) currently provide efficacy in the treatment of major depressive disorder (MDD) and are generally perceived by psychiatrists and primary care physicians as effective, well-tolerated and easily administered. However, they are associated with undesirable features, such as high incidence of sexual dysfunction, delayed onset of action and a level of non-responsiveness estimated to be as high as 30% (see M. J. Gitlin, Journal of Clinical Psychiatry, **1994,** 55, 406-413 and R. T. Segraves, Journal of Clinical Psychiatry, **1992**, 10(2), 4-10). Preclinical and clinical evidence has indicated that the sexual dysfunction associated with SSRI therapy can be reduced through the use of monoamine reuptake inhibitors (SRI) and dopamine reuptake inhibitors (DRIs), such as bupropion (see A. K. Ashton, Journal of Clinical Psychiatry, **1998,** 59(3), 112-115). Furthermore, the combination of SRI and DRI may hasten the onset of action as well as offering relief to refractory patients, possibly through a synergistic mechanism (see R. D. Marshall et al, Journal of Psychopharmacology, **1995,** 9(3), 284-286) and prove beneficial in the treatment of substance abuse and attention deficit hyperactivity disorder (ADHD) according to Barrickman et al, Journal of the American Academy of Child and Adolescent Psychology, **1995,** 34(5), 649 and Shekim et al, Journal of Nervous and Mental Disease, **1989,** 177(5), 296. Their utility in OCD is inferred from D.J. Stein et al., Journal of Clinical Psychiatry, **1997**,58(3), 119.

European patent application EP 0 830 864 discloses combination therapies for the treatment of psychoses, comprising administering to patients a serotonin reuptake inhibitor in combination with an atypical antipsychotic.

PCT application WO 0 024 399 describes pharmaceutical compositions comprising fluoxetine and an antipsychotic agent for use in psychotic disorders, depression and anxiety.

### Summary Of The Invention

The present invention relates to a pharmaceutical composition for the treatment of depression, OCD and psychosis comprising: components (a), (b) and (c) as defined in the appended claims, wherein the active agents "a" and "b" above are present in amounts that render the composition effective in treating, respectively, OCD, psychosis, and depression refractory to treatment with traditional antidepressant therapies alone.

This invention also relates to a method of treating OCD, psychosis and depression In a mammal, comprising administering to said mammal, respectively, an antidepressant, anti-OCD or anti-psychosis effective amount of a pharmaceutical composition as defined above; wherein the active agents "a" and "b" above are present in amounts that render the composition effective in treating, respectively, OCD, psychosis and depression with improvement in the efficacy achieved by either component individually.

This invention also relates to a method of treating OCD, psychosis and depression in a mammal, comprising administering to said mammal: components (a) and (b) as defined; wherein the active agents "a" and "b" above are present in amounts that render the combination of the two agents effective in treating, respectively, OCD, psychosis and depression with improvement in the efficacy achieved by either component individually in the treatment of OCD, depression, especially refractory depression, and psychosis.

It will be appreciated that when using a combination method of the present invention, referred to immediately above, both the atypical antipsychotic agent and the SRI antidepressant agent will be administered to a patient within a reasonable period of time. The compounds may be in the same pharmaceutically acceptable carrier and therefore administered simultaneously. They may be in separate pharmaceutical carriers such as conventional oral dosage forms that are taken simultaneously. The term combination, as used above, also refers to the case where the compounds are provided in separate dosage forms and are administered sequentially. Therefore, by way of example, the SRI antidepressant agent may be administered as a tablet and then, within a reasonable period of time, the atypical antipsychotic agent may be administered either as an oral dosage form such as a tablet or a fast-dissolving oral dosage form. By a "fast dissolving oral formulation" is meant, an oral delivery form which when placed on the tongue of a patient, dissolves within about seconds.

The compositions of the present invention that contain an atypical antipsychotic agent and an SRI antidepressant are useful for the treatment of OCD, depression, especially refractory depression, or psychosis, especially schizophrenia. As used herein, the term "depression" includes depressive disorders, for example, single episodic or recurrent major depressive disorders, and dysthymic disorders, depressive neurosis, and neurotic depression; melancholic depression including anorexia, weight loss, insomnia and early morning waking, and psychomotor retardation; atypical depression (or reactive depression) including increased appetite, hypersomnia, psychomotor agitation or irritability, anxiety and phobias, seasonal affective disorder, or bipolar disorders or manic depression, for example, bipolar I disorder, bipolar II disorder, cyclothymic disorder and OCD.

Other mood disorders encompassed within the term "depression" include dysthymic disorder with early or late onset and with or without atypical features; dementia of the Alzheimer's type, with early or late onset, with depressed mood; vascular dementia with depressed mood, disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood.

The compositions of the present invention are especially useful for the treatment of depression, especially refractory depression where the use of an antidepressant is generally prescribed. By the use of a combination of an atypical antipsychotic agent and an SRI antidepressant agent in accordance with the present invention, it is possible to treat depression, especially refractory depression, in patients for whom conventional antidepressant therapy might not be wholly successful or where OCD or psychosis, especially schizophrenia, are present.

Examples of Serotonin Reuptake Inhibitors (SRI) that can be used in the method and pharmaceutical compositions of this invention are compounds of the formula 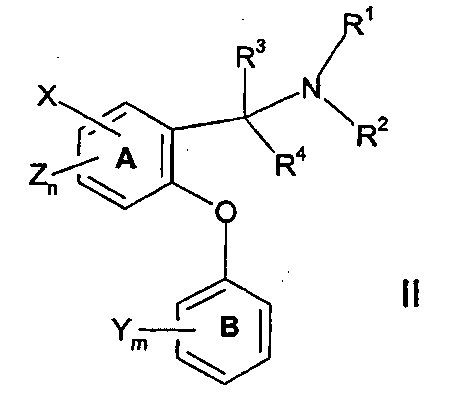 wherein phenyl ring A and phenyl ring B can each, independently, be replaced by a naphthyl group, and wherein when phenyl ring A is replaced by a naphthyl group, the ethereal oxygen of Formula II and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl (e.g., furan, thiophene, pyrrole, thiazole, isothiazole, oxazole, isoxazole, imidazole, 1,2,4-oxadiazole, 1,2,4-thiadiazole, 1,2,4-triazole, 1,2,3,-triazole, tetrazole, pyridine, pyrimidine, pyrazine, quinoline, isoquinoline, quinazoline, quinoxaline, benzothiophene, benzofuran, benzimidazole, benzisoxazole, benzisothiazole and indole) or heterocycle (e.g., imidazolidine, oxazolidine, thiazolidine, pyrrolidine, piperidine, morpholine) groups as defined below and may be further substituted by hydrogen, halo (i.e., fluorine, chlorine, bromine, iodine), (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo (i.e., chloro, fluoro, bromo or iodo), (C₁-C₄)alkyl optionally substituted with from one to three fluorine atoms, (C₁-C₄)alkoxy;
and the pharmaceutically acceptable salts thereof. Compounds of formula II, and their pharmaceutically acceptable salts, have activity in inhibiting reuptake of serotonin, dopamine, and norepinephrine.

In one embodiment, ring B is phenyl, not replaced with a naphthyl group. In another embodiment, phenyl ring B in the compounds of formula II is replaced with a naphthyl group.

In a preferred embodiment when ring B is phenyl, each Y is hydrogen or halo. In a more preferred embodiment, m is 1 or 2, and each Y is chlorine.

In another embodiment, compounds of formula II, or pharmaceutically acceptable salts, thereof are described above, but wherein X is selected from furan, thiophene, pyrrole, and 1,2,3-triazole, and wherein X may be further substituted.

In another embodiment, compounds of formula II or salts thereof are described above, but wherein each Z is selected from hydrogen and halo. Preferably, Z is hydrogen.

In a further embodiment, compounds of formula II or salts thereof are described above, wherein R³ and R⁴ are independently selected from hydrogen and unsubstituted (C₁-C₄) alkyl. Preferably, one or both of R³ and R⁴ are hydrogen.

In a further embodiment, formula II or salts thereof, wherein R¹ and R² are independently selected from hydrogen and unsubstituted (C₁-C₄)alkyl. Preferably, one of R¹ and R² is hydrogen and the other of R¹ and R² is (C₁-C₄)alkyl. More preferably, one of R¹ and R² is hydrogen and the other of R¹ and R² is methyl.

The methods and pharmaceutical compositions of this invention also relates to the pharmaceutically acceptable acid addition salts of the compounds of formula II. Examples of pharmaceutically acceptable acid addition salts of the compounds of formula II are the salts of hydrochloric acid, p-toluenesulfonic acid, fumaric acid, citric acid, succinic acid, salicylic acid, oxalic acid, hydrobromic acid, phosphoric acid, methanesulfonic acid, tartaric acid, maleic acid, di-p-toluoyl tartaric acid, acetic acid, sulfuric acid, hydroiodic acid and mandelic acid.

Unless otherwise indicated, the term "halo", as used herein, includes fluoro, chloro, bromo and iodo.

Unless otherwise indicated, the term "alkyl", as used herein, may be straight, branched or cyclic, and may include straight and cyclic moieties as well as branched and cyclic moieties.

When reference is made to SOₚ(C₁-C₆)alkyl, and p is two, this indicates a sulfone, in other words, S(=O)₂(C₁-C₆)alkyl.

The term "treatment", as used herein, refers to reversing, alleviating, inhibiting the progress of, or preventing the disorder or condition to which such term applies, or one or more symptoms of such condition or disorder. The term "treatment", as used herein, refers to the act of treating, as "treating" is defined immediately above.

When reference is made herein to a disorder or condition that can be treated by inhibiting the reuptake of serotonin, dopamine, or norepinephrine, this means that the disorder or condition has as a contributing factor at least one of serotonin, dopamine, or norepinephrine-mediated neurotransmission. The disorder or condition may have as a contributing factor one, two, or all three of the aforementioned types of neurotransmission. Moreover, a factor or factors other than serotonin, dopamine, or norepinephrine-mediated neurotransmission may also contribute to the disorder or condition. Disorders and conditions to which serotonin, dopamine, or norepinephrine-mediated neurotransmission contribute can be ascertained by those of ordinary skill in the art and include, but are not limited to, for example, addiction and substance abuse, depression, and phobia.

The compounds of formula II may have optical centers and therefore may occur in different enantiomeric configurations. The invention includes all enantiomers, diastereomers, and other stereoisomers of such compounds of formula II, as well as racemic and other mixtures thereof.

Formula II compounds also include isotopically-labeled compounds, which are identical to those recited in formula II, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as ³H, ¹¹C, ¹⁴C, ¹⁸F, ¹²³I and ¹²⁵I. Compounds of the present invention and pharmaceutically acceptable salts of said compounds that contain the aforementioned isotopes and/or other isotopes of other atoms are within the scope of this invention. Isotopically labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances.

"Chemical dependency," as used herein, means an abnormal craving or desire for, or an addiction to a drug. Such drugs are generally administered to the affected individual by any of a variety of means of administration, including oral, parenteral, nasal or by inhalation. Examples of chemical dependencies treatable by the methods of the present invention are dependencies on alcohol, nicotine, cocaine, heroin, Phenobarbital, and benzodiazepines (e.g., Valium (trademark)). "Treating a chemical dependency," as used herein, means reducing or alleviating such dependency.

Preferred embodiments of the compounds of formula II include the following compounds of the formula II and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methylamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethylphenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine;
[2-(3,4-Dichlorophenoxy)-4-thiophen-2-ylbenzyl]-methylamine;
[2-(3,4-Dichlorophenoxy)-5-pyrimidin-2-ylbenzyl]-methylamine;
[2-(3,4-Dichlorophenoxy)-5-pyrimidin-4-ylbenzyl]-methylamine;
[2-(3,4-Dichlorophenoxy)-5-(2-methylpyrimidin-4-yl)-benzyl]-methylamine;
{1-[2-(3,4-Dichlorophenoxy)-5-(2-methylpyrimidin-4-yl)-phenyl]-ethyl}-methylamine;
4-[4-(3,4-Dichlorophenoxy)-3-(1-methylpyrrolidin-2-yl)-phenyl]-2-methylpyrimidine;
[2-(4-Chlorophenoxy)-5-(1-methyl-1H-pyrrol-3-yl)-benzyl]-dimethylamine;
[5-(1-methyl-1H-pyrrol-3-yl)-2-(naphthalen-2-yloxy)-benzyl]-dimethyl amine;
[5-Imidazol-1-yl-2-(naphthalen-2-yloxy)-benzyl]-dimethylamine;
1,5,5-Trimethyl-3-[3-methylaminomethyl-4-(naphthaten-2-yloxy)-phenyl]-imidazolidine-2,4-dione;
1-Methyl-3-[3-methylaminomethyl-4-(naphthalen-2-yloxy)-phenyl]-imidazolidine-2,4-dione;
3-[3-Methylaminomethyl-4-(naphthalen-2-yloxy)-phenyl]-thiazolidine-2,4-dione;
3-[3-Methylaminomethyl-4-(naphthalen-2-yloxy)-phenyl]-oxazolidine-2,4-dione;
3-[3-Methylaminomethyl-4-(naphthalen-2-yloxy)-phenyl]-oxazolidin-2-one;
3-[3-Methylaminomethyl-4-(naphthalen-2-yloxy)-phenyl]-thiazolidin-2-one;
1-Methyl-3-[3-methylaminomethyl-4-(naphthalen-2-yloxy)-phenyl]-imidazolidin-2-one;
1-Methyl-3-[3-methylaminomethyl-4-(naphthalen-2-yloxy)-phenyl]-tetrahydro-pyrimidin-2-one;
1-[4-(3,4-Dichlorophenoxy)-3-methylaminomethyl-phenyl]-3-methyltetrahydropyrimidin-2-one;
1-[4-(3,4-Dichlorophenoxy)-3-methylaminomethyl-phenyl]-3-methylimidazolidin-2-one;
3-[4-(3,4-Dichlorophenoxy)-3-methylaminomethyl-phenyl]-thiazolidin-2-one;
3-[4-(3,4-Dichlorophenoxy)-3-methylaminomethyl-phenyl]-oxazolidin-2-one;
[2-(3,4-Dichlorophenoxy)-5-(2-methylthiazol-4-yl)-benzyl]-methylamine;
[2-(3,4-Dichlorophenoxy)-5-(2-methyloxazol-4-yl)-benzyl]-methylamine;
[2-(3,4-Dichlorophenoxy)-5-(2,5-dimethyloxazol-4-yl)-benzyl]-methylamine;
[2-(3,4-Dichlorophenoxy)-5-(2,5-dimethylthiazol-4-yl)-benzyl]-methylamine;
[2-(3,4-Dichlorophenoxy)-5-(5-methyl-[1,2,4]thiadiazol-3-yl)-benzyl]-methylamine;
[2-(3,4-Dichlorophenoxy)-5-(5-methyl-[1,2,4]oxadiazol-3-yl)-benzyl]-methylamine;
[2-(3,4-Dichlorophenoxy)-5-[1,2,3]oxadiazol-4-yl-benzyl]-methylamine;
[2-(3,4-Dichlorophenoxy)-5-(5-methyl-[1,2,3]thiadiazol-4-yl)-benzyl]-methylamine;
[2-(3,4-Dichlorophenoxy)-5-(2,4-dimethyloxazol-5-yl)-benzyl]-methylamine;
[2-(3,4-Dichlorophenoxy)-5-(2,4-dimethylthiazol-5-yl)-benzyl]-methylamine;
[2-(3,4-Dichlorophenoxy)-5-[1,2,4]triazol-1-ylbenzyl]-methylamine;
[2-(3,4-Dichlorophenoxy)-5-(3-methyl-[1,2,4]triazol-1-yl)-benzyl]-methylamine;
[2-(4-Chlorophenoxy)-5-(3,5-dimethyl-[1,2,4]triazol-1-yl)-benzyl]-methylamine;
[2-(4-Chlorophenoxy)-5-tetrazol-1-ylbenzyl]-methylamine;
[2-(4-Chlorophenoxy)-5-(5-methyltetrazol-1-yl)-benzyl]-dimethylamine;
[2-(4-Chlorophenoxy)-5-[1,2,4]triazol-4-ylbenzyl]-dimethylamine;
[2-(4-Chlorophenoxy)-5-(1-methyl-1H-tetrazol-5-yl)-benzyl]-dimethylamine; and
{1-[2-(3,4-Dichlorophenoxy)-5-(1-methyl-1H-tetrazol-5-yl)-phenyl]-ethyl}-dimethylamine.

Suitable classes of an atypical antipsychotic agent that may be used in the compositions and methods of this invention include the following compounds:
abaperidone
7-[3-[4-(6-fluoro-1,2-benzisoxazol-3-yl)-1-piperidinyl]propoxy]-3-(hydroxymethyl)-4H-1-benzopyran-4-one;
belaperidone
(1α,5α,6α)-3-[2-[6-(4-fluorophenyl)-3-azabicyclo[3.2.0]-hept-3-yl]ethyl]-2,4(1 H,3H)quinazolinedione;
clozapine
8-chloro-11-(4-methyl-1-piperazinyl)-5H-dibenzo[b,e][1,4]diazepine;
iloperidone
1-[4-[3-[4-(6-fluoro-1,2-benzisoxazol-3-yl)-1-piperdinyl]-3-methoxyphenyl]ethanone;
olanzapine
2-methyl-4-(4-methyl-1-piperazinyl)-10H-thieno[2,3-b][1,5]benzodiazepine;
perospirone
cis-2-[4-[4-(1,2-benzisothiazol-3-yl)-1-piperazinyl]butyl]-hexahydro-1H-isoindole-1,3(2H)-dione;
risperidone
3-[2-[4-(6-fluoro-1,2-benzisoxazol-3-yl)piperidino]ethyl]-2-methyl-6,7,8,9-tetrahydro-4H-pyrido[1,2-α]pyrimidin-4-one;
sertindole
1-[2-[4-[5-chloro-1-(4-fluorophenyl-1H-indol-3yl]-1-piperidinyl]ethyl]imidazolidin-2-one;
tiospirone
8-[4-[4-(1,2-benzisothiazol-3-yl)-1-piperazinyl]butyl]-8-azaspiro[4.5]decane-7,9-dione;
ziprasidone
5-[2-[4-(1,2-benzisothiazole-3-yl)-1-piperazinyl]ethyl]-6-chloro-1,3-dihydro-2H-indol-2-one;
zotepine
2-[(8-chlorodibenzo[b,f]thiepin-10-yl)oxy]-N,N-dimethyl-ethanamine;
quetiapine
5-[2-(4-dibenzo[b,f][1,4]thiazepin-11-yl-1-piperazinyl)ethoxy]ethanol; and
blonanserin
2-(4-ethyl-1-piperazinyl)-4-(4-fluorophenyl)-5,6,7,8,9,10-hexahydrocycloocta[b]pyridine.

### Detailed Description Of The Invention

The following references refer to novel biaryl ether derivatives useful as monoamine reuptake inhibitors that exhibit activity as a serotonin reuptake inhibitor and that can be used, in combination with an atypical antipsychotic in the pharmaceutical compositions and methods of this invention, and to methods of preparing the same: PCT application No.: PCT/IB00/01373 Filed Sept 27, 2000 and PCT application No. PCT/IB00/00108 filed Feb 2, 2000. United States Patent No. 4,018,830, issued April 19, 1997, refers to phenylthioaralkylamines and 2-phenylthiobenzylamines which are active as antiarrhythmics.

WO 97/17325, International Publication Date May 15, 1997, refers to derivatives of N,N-dimethyl-2-(arylthio)benzylamine which selectively influence serotonin transport in the central nervous system and are useful as antidepressants.

United States Patent 5,190,965, issued March 2, 1993, and United States Patent 5,430,063, issued July 4, 1995, refer to phenoxyphenyl derivatives which have utility in the treatment of depression.

United States Patent 4,161,529, issued July 17, 1979, refers to pyrrolidine derivatives that possess anticholesteremic and hypolipemic activity.

United States Provisional Application No. 60/121313, filed February 23, 1999, refers to biaryl ethers that have activity in inhibiting reuptake of both serotonin and dopamine.

The SRI antidepressants of the formula I can be prepared as described in the following patent application, which is referred to above: PCT application NO. PCT/IB00/01373 filed September 27, 2000. SRI antidepressants of Formula II can be prepared as described in the following patent application, which is referred to above: PCT application No. PCT/IB00/00108 filed February 2, 2000.

The atypical antipsychotic agents that can be used, together with an SRI antidepressant agent in the pharmaceutical compositions and methods of this invention are those compounds and pharmaceutically acceptable salts described in the following references:
abaperidone
7-[3-[4-(6-fluoro-1,2-benzisoxazol-3-yl)-1-piperidinyl]propoxy]-3-(hydroxymethyl)-4H-1-benzopyran-4-one; United States Patent No. 5,736,588 issued April 7, 1998;
belaperidone
(1 α,5α,6α)-3-[2-[6-(4-fluorophenyl)-3-azabicyclo[3.2.0]-hept-3-yl]ethyl]-2,4(1H,3H)quinazolinedione; United States Patent No. 5,475,105 issued December 12, 1995;
clozapine
(8-chloro-11-(4-methyl-1-piperazinyl)-5H-dibenzo[b,e][1,4]diazepine; United States Patent No. 3,539,573 issued November 10, 1970;
iloperidone
1-[4-[3-[4-(6-fluoro-1,2-benzisoxazol-3-yl)-1-piperdinyl]-3-methoxyphenyl]ethanone; EP-402,644 granted December 19, 1990;
olanzapine
2-methyl-4-(4-methyl-1-piperazinyl)-10H-thieno[2,3-b][1,5]benzodiazepine; United States Patent No. 5,229,382 issued July 20, 1993;
perospirone
cis-2-[4-[4-(1,2-benzisothiazol-3-yl)-1-piperazinyl]butyl]-hexahydro-1H-isoindole-1,3(2H)-dione; United States Patent No. 4,745,117 issued May 17, 1988;
risperidone
3-[2-[4-(6-fluoro-1,2-benzisoxazol-3-yl)piperidino]ethyl]-2-methyl-6,7,8,9-tetrahydro-4H-pyrido[1,2-α]pyrimidin-4-one; United States Patent No. 4,804,663 issued February 14, 1989;
sertindole
1-[2-[4-[5-chloro-1-(4-fluorophenyl-1H-indol-3yl]-1-piperidinyl]ethyl]imidazolidin-2-one; United States Patent No. 4,710,500; 5,112,838; and 5,238,945 that were issued December 1, 1987, May 12, 1992, August 24, 1992;
tiospirone
8-[4-[4-(1,2-benzisothiazol-3-yl)-1-piperazinyl]butyl]-8-azaspiro[4.5]decane-7,9-dione; United States Patent No. 4,411,901 issued October 25, 1983;
ziprasidone
5-[2-[4-(1,2-benzoisothiazole-3-yl)-1-piperazinyl]ethyl]-6-chloro-1,3-dihydro-2H-indol-2-one; United States Patent No. 4,831,031 issued May 16, 1989;
zotepine
2-[(8-chlorodibenzo[b,f]thiepin-10-yl)oxy]-N,N-dimethyl-ethanamine; United States Patent No. 3,704,245 issued November 28, 1972;
quetiapine
5-[2-(4-dibenzo[b,f][1,4]thiazepin-11-yl-1piperazinyl)ethoxy]ethanol; United States Patent No. 4,879,288 issued November 7, 1989; and
blonanserin
2-(4-ethyl-1-piperazinyl)-4-(4-fluorophenyl)-5,6,7,8,9,10-hexahydrocycloocta[b]pyridine; United States Patent No. 5,021,421 issued June 4, 1991.

This invention relates both to uses in depression, obsessive compulsive disorder (OCD) and psychosis in which the atypical antipsychotic agent and the SRI antidepressant agent, or pharmaceutically acceptable salts of the same, are administered together, as part of the same pharmaceutical composition, as well as to uses in which these two active agents are administered separately as part of an appropriate dose regimen designed to obtain the benefits of the combination therapy. The appropriate dose regimen, the amount of each dose administered, and specific intervals between doses of each active agent will depend upon the subject being treated and the severity of the condition. Generally, in carrying out the methods of this invention, the atypical antipsychotic agent will be administered to an adult human in an amount ranging from 0.05 to 1500 mg per day, in single or divided doses, preferably from 5 to 200 mg/day. The compounds may be administered on a regimen of up to 6 times per day, preferably 1 to 4 times per day, especially 2 times per day and most especially once daily. A suitable dosage level for the SRI antidepressant agent is about 0.5 to 1500 mg per day, preferably about 2.5 to 1000 mg per day, and especially about 2.5 to 500 mg per day. The compounds may be administered on a regimen of up to 6 times per day, preferably 1 to 4 times per day, especially 2 times per day and most especially once dailyVariations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The atypical antipsychotic and their pharmaceutically acceptable salts, and the SRI antidepressant agents and their pharmaceutically acceptable salts that are employed in the pharmaceutical compositions and methods of this invention are hereinafter also referred to as "therapeutic agents". The therapeutic agents can be administered via either the oral or parenteral route. Compositions containing both an atypical antipsychotic agent and an SRlantidepressant agent, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i*.*e*., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, *etc*. Moreover, oral pharmaceutical compositions can be suitably sweetened and/or flavored. In general, the therapeutic agents of this invention, when administered separately (*i*.*e*., not in the same pharmaceutical composition) are present in such dosage forms at concentration levels ranging from 5.0% to 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intra-articular, intramuscular and subcutaneous injection purposes. The preparation of all these solutions under sterile conditions is readily accomplished by standard pharmaceutical techniques well known to those skilled in the art.

As stated above, the atypical antipsychotic agent and the SRI antidepressant agent may be formulated in a single pharmaceutical composition or alternatively in individual pharmaceutical compositions for simultaneous, separate or sequential use in accordance with the present invention.

Preferably the compositions according to the present invention, which contain both an atypical antipsychotic and an SRI antidepressant, as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, e.g., conventional tableting ingredients such as com starch, lactose, sucrose, sorbitol, talc, stearic acid, magnesium stearate, dicalcium phosphate or gums, and other pharmaceutical diluents, *e.g*., water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from 0.05 to about 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration of an atypical antipsychotic agent or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g.*, Tween™ 20, 40, 60, 80 or 85) and other sorbitans (*e*.*g*., Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn ™, Infonutrol™, Lipofundin ™ and Lipiphysan™. The therapeutic agent may be either dissolved in a pre-mixed emulsion composition or alternatively it may be dissolved in an oil (*e.g.*, soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable, aqueous or organic solvents or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising an atypical antipsychotic agent and an SRI antidepressant, or pharmaceutically acceptable salts of the same, which process comprises bringing an atypical antipsychotic agent and the SRI antidepressant agent ,or the pharmaceutically acceptable salts, into association with a pharmaceutically acceptable carrier or excipient.

It will be appreciated that the amount of the atypical antipsychotic agent and the SRI antidepressant agent required for use in the treatment of depression will vary not only with the particular compounds or compositions selected but also with the route of administration, the nature of the condition being treated, and the age and condition of the patient, and will ultimately be at the discretion of the patient's physician or pharmacist.

The *in vitro* activity of the SRI compounds used in this invention at the individual monoamine reuptake sites can be determined using rat synaptosomes or HEK-293 cells transfected with the human serotonin, dopamine or norepinephrine transporter, according to the following procedure adapted from those described by S. Snyder et al., (Molecular Pharmacology, **1971,** 7, 66-80), D.T. Wong et al., (Biochemical Pharmacology, **1973,** 22, 311-322), H. F. Bradford (Journal of Neurochemistry, **1969,** 16, 675-684) and D. J. K. Balfour (European Journal of Pharmacology, **1973,** 23, 19-26).

Synaptosomes: Male Sprague Dawley rats are decapitated and the brains rapidly removed. The cortex, hippocampi and corpus striata are dissected out and placed in ice cold sucrose buffer, 1 gram in 20 ml of buffer (the buffer is prepared using 320 mM sucrose containing 1mg/ml glucose, 0.1mM ethylenediamine tetraacetic acid (EDTA) adjusted to pH 7.4 with tris(hydroxymethyl)-aminomethane (TRIS) base). The tissues are homogenized in a glass homogenizing tube with a Teflon™ pestle at 350 rpm using a Potters homogenizer. The homogenate is centrifuged at 1000 x g for 10 min. at 4°C. The resulting supernatant is recentrifuged at 17,000 x g for 20 min. at 4°C. The final pellet is resuspended in an appropriate volume of sucrose buffer that yielded less than 10% uptake.

Cell Preparation: HEK-293 cells transfected with the human serotonin (5-HT), norepinephrine (NE) or dopamine (DA) transporter are grown in DMEM (Dulbecco's Modified Eagle Medium, Life Technologies Inc., 9800 Medical Center Dr., Gaithersburg, MD, catalog no. 11995-065)) supplemented with 10% dialyzed FBS (Fetal Bovine Serum, from Life Technologies, catalog no. 26300-053), 2 mM L-glutamine and 250 ug/ml G418 for the 5-HT and NE transporter or 2ug/ml puromycin for the DA transporter, for selection pressure. The cells are grown in Gibco triple flasks, harvested with Phosphate Buffered Saline (Life Technologies, catalog no. 14190-136) and diluted to an appropriate amount to yield less than 10% uptake.

Neurotransmitter Uptake Assay: The uptake assays are conducted in glass tubes containing 50 uL of solvent, inhibitor or 10uM sertraline, desipramine or nomifensine for the 5-HT, NE or DA assay nonspecific uptake, respectively. Each tube contains 400 uL of [3H]5-HT (5 nM final), [3H]NE (10 nM final) or [3H]DA (5 nM final) made up in modified Krebs solution containing 100 uM pargyline and glucose (1mg/ml). The tubes are placed on ice and 50 uL of synaptosomes or cells is added to each tube. The tubes are then incubated at 37° C for 7 min. (5-HT, DA) or 10 min. (NE). The incubation is terminated by filtration (GF/B filters), using a 96-well Brandel Cell Harvester, the filters are washed with modified Krebs buffer and counted using either a Wallac Model 1214 or Wallac Beta Plate Model 1205 scintillation counter.

Determination of the *in vivo* serotonin reuptake inhibition activity and potency of action for the compounds of the present invention can be made by measuring the ability of the compound to block the depletion of serotonin in the anterior cortex induced by (+/-)-para-chloroamphetamine (PCA) in the rat, according to a procedure adapted from R. W. Fuller, H. D. Snoddy and M. L. Cohen in Neuropharmacology 23: 539-544 (1984).

Generally, male, white Sprague-Dawley rats weighing 160-230 g each are assigned to either the control (vehicle) or test groups. When the test compound is administered subcutaneously (sc) at a given dose, it is co-administered with 5 mg/kg of para-chloroamphetamine (PCA). Three hours post-dose, the animals are sacrificed by decapitation and the anterior cortices are removed, wrapped in parafilm and frozen in dry ice (-78 C). When dosed orally (po), the rats are fasted the night before the experiment and then treated with the test compound at a given dose 30 minutes prior to the administration of the PCA (5 mg/kg, sc). After three hours, the animals are sacrificed and the tissues removed as above.

To determine the serotonin (5-HT) levels, the frozen tissues are homogenized with Branson sonifier in 0.5 mL of mobile phase in Eppendorf centrifuge tubes. Samples are then spun down at 11000 rpm for twenty minutes in a Sorval SH-MT rotor in a Sorval RC5C centrifuge. The supernatant thus obtained is pipetted into HPLC vials and the 5-HT levels are measured on HPLC-EC.

Interpretation of the results is as follows: Each experiment has a set of vehicle treated animals and a set of PCA-only animals. The mean 5-HT value of the PCA animals is subtracted from the mean 5-HT value of the vehicle animals. This is the signal or window of the response. The mean 5-HT value of each test group is determined, the mean of the PCA group subtracted from that, and that amount divided by the window is the per cent protection from the PCA effect for that dose. To report an ID₅₀, a line is drawn mathematically through the per cent protection values and the 50 per cent level calculated.

All of the title compounds of Formula I and II were assayed *in vitro* for serotonin, dopamine, and norepinephrine reuptake inhibition, and all had IC₅₀ values of about less than or equal to 250 nM for serotonin reuptake inhibition, about less than or equal to 1000 nM for dopamine reuptake inhibition, and about less than or equal to 1000 nM for norepinephrine reuptake inhibition.

When administered in combination, either as a single or as separate pharmaceutical composition(s), the atypical antipsychotic agent and an SRI antidepressant agent, are presented in a ratio which is consistent with the manifestation of the desired effect. In particular, the ratio by weight of the atypical antipsychotic agent and the SRI antidepressant agent will suitably be between 0.001 to 1 and 1000 to 1, and especially between 0.01 to I and 100 to 1.

As used herein the term "mammal" includes animals of economic importance such as bovine, ovine, and porcine animals, especially those that produce meat, as well as domestic animals (*e.g.* cats and dogs), sports animals (*e.g.* horses), zoo animals, and humans, the latter being preferred.

## Claims

1. A pharmaceutical composition for the treatment of depression, obsessive compulsive disorder and psychosis in a mammal, comprising: (a) a compound that exhibits activity, respectively, as an SRI antidepressant, or a pharmaceutically acceptable salt thereof; (b) atypical antipsychotic agent or pharmaceutically acceptable salt thereof; and (c) a pharmaceutically acceptable carrier; wherein the active agents "a" and "b" above are present in amounts that render the composition effective in treating, respectively, anxiety or depression, obsessive compulsive disorder and psychosis with improved efficacy, and wherein the SRI antidepressant agent or pharmaceutically acceptable salt thereof is selected from compounds of the formula II, as defined below, and their pharmaceutically acceptable salts: 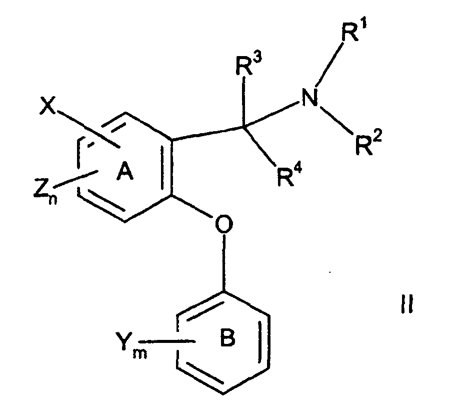 wherein phenyl ring A and phenyl ring B can each, independently, be replaced by a naphthyl group, and wherein when phenyl ring A is replaced by a naphthyl group, the ethereal oxygen of formula II and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four- to eight-membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four- to eight-membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four- to eight-membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl and heterocycle, and wherein each X may be further substituted by hydrogen, halo, (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo, (C₁-C₄)alkyl optionally substituted with from one to three fluorine atoms, (C₁-C₄)alkoxy;
or a pharmaceutically acceptable salt thereof.

2. A pharmaceutical composition according to claim 1 wherein the atypical antipsychotic agent or a pharmaceutically acceptable salt thereof is selected from:
abaperidone
7-[3-[4-(6-fluoro-1,2-benzisoxazol-3-yl)-1-piperidinyl]propoxy]-3-(hydroxymethyl)-4H-1-benzopyran-4-one;
belaperidone
(1a,5a,6a)-3-[2-[6-(4-fluorophenyl)-3-azabicyclo[3.2.0]-hept-3-yl]ethyl]-2,4(1 H,3H)quinazolinedione;
clozapine
8-chloro-11-(4-methyl-1-piperazinyl)-5H-dibenzo[b,e][1,4]diazepine
iloperidone
1-[4-[3-[4-(6-fluoro-1,2-benzisoxazol-3-yl)-1-piperdinyl]-3-methoxyphenyl]ethanone;
olanzapine
2-methyl-4-(4-methyl-1-piperazinyl)-10H-thieno[2,3-b][1,5]benzodiazepine;
perospirone
cis-2-[4-[4-(1,2-benzisothiazol-3-yl)-1-piperazinyl]butyl]-hexahydro-1H-isoindole-1,3(2H)-dione;
risperidone
3-[2-[4-(6-fluoro-1,2-benzisoxazol-3-yl)piperidino]ethyl]-2-methyl-6,7,8,9-tetrahydro-4H-pyrido[1,2-α]pyrimidin-4-one;
sertindole
1-[2-[4-[5-chloro-1-(4-fluorophenyl-1H-indol-3yl]-1-piperidinyl]ethyl]imidazolidin-2-one;
tiospirone
8-[4-[4-(1,2-benzisothiazol-3-yl)-1-piperazinyl]butyl]-8-azaspiro[4,5]decane-7,9-dione;
ziprasidone
5-[2-[4-(1,2-benzisothiazole-3-yl)-1-piperazinyl]ethyl]-6-chloro-1,3-dihydro-2H-indol-2-one;
zotepine
2-[(8-chlorodibenzo[b,f]thiepin-10-yl)oxy]-N,N-dimethyl-ethanamine;
quetiapine
5-[2-(4-dibenzo[b,f][1,4]thiazepin-11-yl-1piperazinyl)ethoxy]ethanol; and
blonanserin
2-(4-ethyl-1-piperazinyl)-4-(4-fluorophenyl)-5,6,7,8,9,10-hexahydrocycloocta[b]pyridine.

3. A pharmaceutical composition according to Claim 1 wherein the SRI antidepressant, or pharmaceutically acceptable salt thereof, is present in an amount of from 0.05 mg to 1500 mg and the atypical antipsychotic agent, or pharmaceutically acceptable salt thereof, is present in an amount of from 0.5 mg to 1500 mg.

4. The use of a pharmaceutical composition according to any of claims 1 to 3, in the manufacture of a medicament for the treatment of anxiety or depression, obsessive compulsive disorder, and psychosis.

5. Use according to Claim 4, wherein the SRI antidepressant, or pharmaceutically acceptable salt thereof, is present in said medicament in an amount of from 0.05 mg to 1500 mg, and the atypical antipsychotic agent, or pharmaceutically acceptable salt thereof, is present in an amount of from 0.05 mg to 1500 mg.

6. The use of an SRI antidepressant, or a pharmaceutically acceptable salt thereof, in the manufacture of a medicament for the treatment of anxiety or depression, obsessive compulsive disorder, and psychosis for administration to patients simultaneously, separately or sequentially receiving an atypical antipsychotic or pharmaceutically acceptable salt thereof, wherein the SRI antidepressant or pharmaceutically acceptable salt thereof is selected from compounds of the formula II, 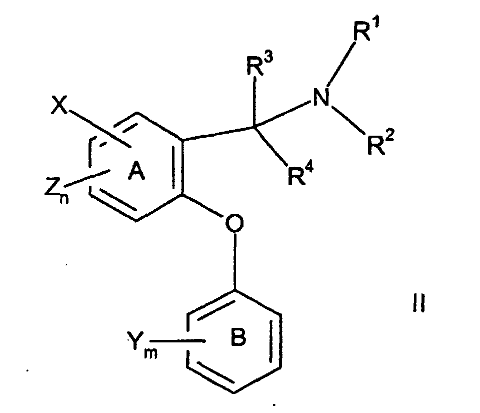 wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four- to eight-membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four- to eight-membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four- to eight-membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl and heterocycle, and wherein each X may be further substituted by hydrogen, halo, (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo, (C₁-C₄)alkyl optionally substituted with from one to three fluorine atoms, (C₁-C₄)alkoxy;
or a pharmaceutically acceptable salt thereof.

7. Use according to Claim 6, wherein the SRI antidepressant, or pharmaceutically acceptable salt thereof, is present in said medicament in an amount of from 0.05 mg to 1500 mg, and the atypical antipsychotic agent, or pharmaceutically acceptable salt thereof, is administered to the patient in an amount of from 0.05 mg day to 1500 mg per day.

8. Use according to Claim 4 or 6, wherein the atypical antipsychotic agent or pharmaceutically acceptable salt thereof is selected from:
abaperidone
7-[3-[4-(6-fluoro-1,2-benzisoxazol-3-yl)-1-piperidinyl]propoxy]-3-(hydroxymethyl)-4H-1-benzopyran-4-one;
belaperidone
(1a,5a,6a)-3-[2-[6-(4-fluorophenyl)-3-azabicyclo[3.2.0]-hept-3-yl]ethyl]-2,4(1 H,3H)quinazolinedione;
clozapine
8-chloro-11-(4-methyl-1-piperazinyl)-5H-dibenzo[b,e][1,4]diazepine;
iloperidone
1-[4-[3-[4-(6-fluoro-1,2-benzisoxazol-3-yl)-1-piperdinyl]-3-methoxyphenyl]ethanone;
olanzapine
2-methyl-4-(4-methyl-1-piperazinyl)-10H-thieno[2,3-b][1,5]benzodiazepine;
perospirone
cis-2-[4-[4-(1,2-benzisothiazol-3-yl)-1-piperazinyl]butyl]-hexahydro-1H-isoindole-1,3(2H)-dione;
risperidone
3-[2-[4-(6-fluoro-1,2-benzaisoxazol-3-yl)piperidino]ethyl]-2-methyl-6,7,8,9-tetrahydro-4H-pyrido[1,2-α]pyrimidin-4-one;
sertindole
1-[2-[4-[5-chloro-1-(4-fluorophenyl-1H-indol-3yl]-1-piperidinyl]ethyl]imidazolidin-2-one; tiospirone
8-[4-[4-(1,2-benzisothiazol-3-yl)-1-piperazinyl]butyl]-8-azaspiro[4.5]decane-7,9-dione; ziprasidone
5-[2-[4-(1,2-benzisothiazole-3-yl)-1-piperazinyl]ethyl]-6-chloro-1m3-dihydro-2H-indol-2-one;
zotepine
2-[(8-chlorodibenzo[b,f]thiepin-10-yl)oxy]-N,N-dimethyl-ethanamine;
quetiapine
5-[2-(4-dibenzo[b,f][1,4]thiazepin-17-yl-1piperaz-inyl)ethoxy]ethanol; and
blonanserin
2-(4-ethyl-1-piperazinyl)-4-(4-fluorophenyl)-5,6,7,8,9,10-hexahydrocycloocta[b]pyridine.

## Patentansprüche

1. Pharmazeutische Zusammensetzung für die Behandlung von Depression, zwangsneurotischen Störungen und Psychose bei einem Säuger, umfassend
(a) eine Verbindung, die Aktivität als SRI-Antidepressivum aufweist, oder ein pharmazeutisch annehmbares Salz davon;
(b) ein atypisches Antipsychotikum oder ein pharmazeutisch annehmbares Salz davon; und
(c) einen pharmazeutisch annehmbaren Träger, wobei die Wirkstoffe "a" und "b" oben in Mengen vorliegen, die die Zusammensetzung für eine Behandlung von Angst oder Depression, zwangsneurotischen Störungen und Psychose mit verbesserter Wirksamkeit wirksam machen, und wobei das SRI-Antidepressivum oder das pharmazeutisch annehmbare Salz davon aus Verbindungen der Formel II, wie sie unten definiert ist, und deren pharmazeutisch annehmbaren Salzen ausgewählt ist: 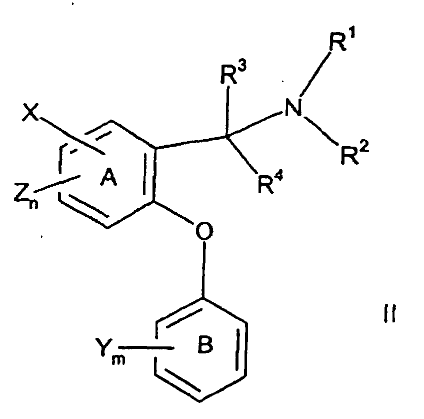
worin Phenylring A und Phenylring B jeweils unabhängig voneinander durch eine Naphthylgruppe ersetzt sein können und, wenn Phenylring A durch eine Naphthylgruppe ersetzt ist, der Ethersauerstoff der Formel II und der Kohlenstoff, an den R³, R⁴ und NR¹R² gebunden sind, an benachbarten Ringkohlenstoffatomen der Naphthylgruppe gebunden sind und keines der benachbarten Ringkohlenstoffatome auch einem Kohlenstoffatom des kondensierten Rings der Naphthylgruppe benachbart ist;
n und m unabhängig aus eins, zwei und drei ausgewählt sind;
R¹ und R² unabhängig aus Wasserstoff, (C₁-C₄)Alkyl, (C₂-C₄)Alkenyl und (C₂-C₄)Alkinyl ausgewählt sind oder R¹ und R² zusammen mit dem Stickstoffatom, an das sie gebunden sind, einen vier- bis achtgliedrigen gesättigten Ring bilden, der ein oder zwei Heteroatome, einschließlich des Stickstoffs, an den R¹ und R² gebunden sind, enthält, wobei das zweite Heteroatom, wenn es vorliegt, aus Sauerstoff, Stickstoff und Schwefel ausgewählt ist, und wobei der Ring an verfügbaren Bindungsstellen gegebenenfalls mit ein bis drei Substituenten substituiert sein kann, welche unabhängig voneinander aus Hydroxy und (C₁-C₆)Alkyl ausgewählt sind;
R³ und R⁴ unabhängig voneinander aus Wasserstoff und (C₁-C₄)Alkyl, das gegebenenfalls mit ein bis drei Fluoratomen substituiert ist, ausgewählt sind oder R³ und R⁴ zusammen mit dem Kohlenstoff, an den sie gebunden sind, einen vier- bis achtgliedrigen gesättigten carbocyclischen Ring bilden und wobei der Ring an verfügbaren Bindungsstellen gegebenenfalls mit ein bis drei Substituenten substituiert sein kann, welche unabhängig aus Hydroxy und (C₁-C₆)Alkyl ausgewählt sind;
oder R² und R³ zusammen mit dem Stickstoff, an den R² gebunden ist, und dem Kohlenstoff, an den R³ gebunden ist, einen vierbis achtgliedrigen gesättigten Ring bilden, der ein oder zwei Heteroatome, einschließlich des Stickstoffs, an den R² gebunden ist, enthält, wobei das zweite Heteroatom, wenn es vorhanden ist, aus Sauerstoff, Stickstoff und Schwefel ausgewählt ist, und wobei der Ring gegebenenfalls an verfügbaren Bindungsstellen mit einem bis drei Substituenten substituiert sein kann, welche unabhängig aus Hydroxy und (C₁-C₆)Alkyl ausgewählt sind;
jedes X unabhängig aus Phenyl, Heteroaryl und Heterocyclus ausgewählt ist und wobei jedes X außerdem durch Wasserstoff, Halogen, (C₁-C₄)Alkyl, das gegebenenfalls mit ein bis drei Fluoratomen substituiert ist, (C₁-C₄)Alkoxy, das gegebenenfalls mit ein bis drei Fluoratomen substituiert ist, Cyano, Nitro, Amino, Hydroxy, Carbonyl, (C₁-C₄)Alkylamino, Di-[(C₁-C₄)alkyl]amino, NR⁵(C=O)(C₁-C₄)Alkyl, SO₂NR⁵R⁶ und SOₚ(C₁-C₆)Alkyl, worin R⁵ und R⁶ unabhängig voneinander aus Wasserstoff und (C₁-C₆)Alkyl ausgewählt sind, und p null, eins oder zwei ist, substituiert sein kann;
jedes Y unabhängig aus Wasserstoff, Halogen, (C₁-C₄)Alkyl, das gegebenenfalls mit ein bis drei Fluoratomen substituiert ist, (C₁-C₄)Alkoxy, das gegebenenfalls mit ein bis drei Fluoratomen substituiert ist, Cyano, Nitro, Amino, (C₁-C₄)Alkylamino, Di-[(C₁-C₄)alkyl]amino, NR⁵(C=O) (C₁-C₄)Alkyl, SO₂NR⁵R⁶ und SOₚ(C₁-C₆)Alkyl, worin R⁵ und R⁶ unabhängig aus Wasserstoff und (C₁-C₆)Alkyl ausgewählt sind und p null, eins oder zwei ist, ausgewählt ist; und
jedes Z unabhängig aus Wasserstoff, Halogen, (C₁-C₄)Alkyl, das gegebenenfalls mit ein bis drei Fluoratomen substituiert ist, (C₁-C₄)Alkoxy ausgewählt ist;
oder ein pharmazeutisch annehmbares Salz.

2. Pharmazeutische Zusammensetzung nach Anspruch 1, wobei das atypische Antipsychotikum oder ein pharmazeutisch annehmbares Salz ausgewählt ist aus:
Abaperidon
7-[3-[4-(6-Fluor-1,2-benzisoxazol-3-yl)-1-piperidinyl]propoxy]-3-(hydroxymethyl)-4H-1-benzopyran-4-on;
Belaperidon
(1a,5a,6a)-3-[2-[6-(4-Fluorphenyl)-3-azabicyclo[3.2.0]-hept-3-yl]ethyl]-2,4(1H,3H)chinazolindion;
Clozapin
8-Chlor-11-(4-methyl-1-piperazinyl)-5H-dibenzo[b,e][1,4]diazepin;
Iloperidon
1-[4-[3-[4-(6-Fluor-1,2-benzisoxazol-3-yl)-1-piperdinyl]-3-methoxyphenyl]ethanon;
Olanzapin
2-Methyl-4-(4-methyl-1-piperazinyl)-10H-thieno[2,3-b][1,5]-benzodiazepin;
Perospiron
Cis-2-[4-[4-(1,2-benzisothiazol-3-yl)-1-piperazinyl]butyl]-hexahydro-1H-isoindol-1,3(2H)-dion;
Risperidon
3-[2-[4-(6-Fluor-1,2-benzisoxazol-3-yl)piperidin]ethyl]-2-methyl-6,7,8,9-tetrahydro-4H-pyrido[1,2-α]pyrimidin-4-on;
Sertindol
1-[2-[4-[5-Chlor-1-(4-fluorphenyl-1H-indol-3yl]-1-piperidinyl]ethyl]imidazolidin-2-on;
Tiospiron
8-[4-[4-(1,2-Benzisothiazol-3-yl)-1-piperazinyl]butyl]-8-azaspiro[4.5]decan-7,9-dion;
Ziprasidon
5-[2-[4-(1,2-Benzisothiazol-3-yl)-1-piperazinyl]ethyl]-6-chlor-1,3-dihydro-2H-indol-2-on;
Zotepin
2-[(8-Chlordibenzo[b,f]thiepin-10-yl)oxy]-N,N-dimethyl-ethanamin;
Quetiapin
5-[2-(4-Dibenzo[b,f][1,4]thiazepin-11-yl-1piperazinyl)ethoxy]ethanol; und
Blonanserin
2-(4-Ethyl-1-piperazinyl)-4-(4-fluorphenyl)-5,6,7,8,9,10-hexahydrocycloocta[b]pyridin.

3. Pharmazeutische Zusammensetzung nach Anspruch 1, wobei das SRI-Antidepressivum oder das pharmazeutisch annehmbare Salz davon in einer Menge von 0,05 mg bis 1500 mg vorliegt und das atypische Antipsychotikum oder das pharmazeutisch annehmbare Salz in einer Menge von 0,5 mg bis 1500 mg vorliegt.

4. Verwendung einer pharmazeutischen Zusammensetzung nach einem der Ansprüche 1 bis 3 bei der Herstellung eines Medikaments zur Behandlung von Angst oder Depression, zwangsneurotischen Störungen und Psychose.

5. Verwendung nach Anspruch 4, wobei das SRI-Antidepressivum oder das pharmazeutisch annehmbare Salz davon in dem Medikament in einer Menge von 0,05 mg bis 1500 mg vorliegt und das atypische Antipsychotikum oder das pharmazeutisch annehmbare Salz davon in einer Menge von 0,05 mg bis 1500 mg vorliegt.

6. Verwendung eines SRI-Antidepressivums oder eines pharmazeutisch annehmbaren Salzes davon in der Herstellung eines Medikaments zur Behandlung von Angst oder Depression, zwangsneurotischen Störungen und Psychose zur gleichzeitigen, getrennten oder aufeinanderfolgenden Verabreichung an Patienten, die ein atypisches Antipsychotikum oder pharmazeutisch annehmbares Salz einnehmen, wobei das SRI-Antidepressivum oder pharmazeutisch annehmbare Salz davon aus Verbindungen der Formel II ausgewählt ist 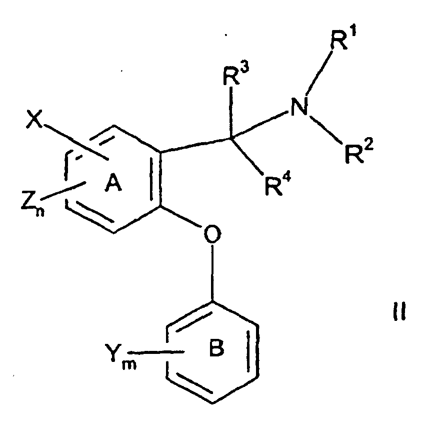 worin Phenylring A und Phenylring B jeweils unabhängig voneinander durch eine Naphthylgruppe ersetzt sein können und, wenn Phenylring A durch eine Naphthylgruppe ersetzt ist, der Ethersauerstoff der Formel I und der Kohlenstoff, an den R³, R⁴ und NR¹R² gebunden sind, an benachbarten Ringkohlenstoffatomen der Naphthylgruppe gebunden sind und keines der benachbarten Ringkohlenstoffatome auch einem Kohlenstoffatom des kondensierten Rings der Naphthylgruppe benachbart ist;
n und m unabhängig aus eins, zwei und drei ausgewählt sind;
R¹ und R² unabhängig aus Wasserstoff, (C₁-C₄)Alkyl, (C₂-C₄)Alkenyl und (C₂-C₄)Alkinyl ausgewählt sind oder R¹ und R² zusammen mit dem Stickstoffatom, an das sie gebunden sind, einen vier- bis achtgliedrigen gesättigten Ring bilden, der ein oder zwei Heteroatome, einschließlich des Stickstoffs, an den R¹ und R² gebunden sind, enthält, wobei das zweite Heteroatom, wenn es vorliegt, aus Sauerstoff, Stickstoff und Schwefel ausgewählt ist, und wobei der Ring an verfügbaren Bindungsstellen gegebenenfalls mit ein bis drei Substituenten substituiert sein kann, welche unabhängig voneinander aus Hydroxy und (C₁-C₆)Alkyl ausgewählt sind;
R³ und R⁴ unabhängig voneinander aus Wasserstoff und (C₁-C₄)Alkyl, das gegebenenfalls mit ein bis drei Fluoratomen substituiert ist, ausgewählt sind oder R³ und R⁴ zusammen mit dem Kohlenstoff, an den sie gebunden sind, einen vier- bis achtgliedrigen gesättigten carbocyclischen Ring bilden und wobei der Ring an verfügbaren Bindungsstellen gegebenenfalls mit ein bis drei Substituenten substituiert sein kann, welche unabhängig aus Hydroxy und (C₁-C₆)Alkyl ausgewählt sind;
oder R² und R³ zusammen mit dem Stickstoff, an den R² gebunden ist, und dem Kohlenstoff, an den R³ gebunden ist, einen vierbis achtgliedrigen gesättigten Ring bilden, der ein oder zwei Heteroatome, einschließlich des Stickstoffs, an den R² gebunden ist, enthält, wobei das zweite Heteroatom, wenn es vorhanden ist, aus Sauerstoff, Stickstoff und Schwefel ausgewählt ist, und wobei der Ring gegebenenfalls an verfügbaren Bindungsstellen mit einem bis drei Substituenten substituiert sein kann, welche unabhängig aus Hydroxy und (C₁-C₆)Alkyl ausgewählt sind;
jedes X unabhängig aus Phenyl, Heteroaryl und Heterocyclus ausgewählt ist und wobei jedes X außerdem durch Wasserstoff, Halogen, (C₁-C₄)Alkyl, das gegebenenfalls mit ein bis drei Fluoratomen substituiert ist, (C₁-C₄)Alkoxy, das gegebenenfalls mit ein bis drei Fluoratomen substituiert ist, Cyano, Nitro, Amino, Hydroxy, Carbonyl, (C₁-C₄)Alkylamino, Di-[(C₁-C₄)alkyl]amino, NR⁵(C=O)(C₁-C₄)Alkyl, SO₂NR⁵R⁶ und SOₚ(C₁-C₆)Alkyl, worin R⁵ und R⁶ unabhängig voneinander aus Wasserstoff und (C₁-C₆)Alkyl ausgewählt sind, und p null, eins oder zwei ist, substituiert sein kann;
jedes Y unabhängig aus Wasserstoff, Halogen, (C₁-C₄)Alkyl, das gegebenenfalls mit ein bis drei Fluoratomen substituiert ist, (C₁-C₄)Alkoxy, das gegebenenfalls mit ein bis drei Fluoratomen substituiert ist, Cyano, Nitro, Amino, (C₁-C₄)Alkylamino, Di- [(C₁-C₄)alkyl]amino, NR⁵(C=O)(C₁-C₄)Alkyl, SO₂NR⁵R⁶ und SOₚ(C₁-C₆)Alkyl, worin R⁵ und R⁶ unabhängig aus Wasserstoff und (C₁-C₆)Alkyl ausgewählt sind und p null, eins oder zwei ist, ausgewählt ist; und
jedes Z unabhängig aus Wasserstoff, Halogen, (C₁-C₄)Alkyl, das gegebenenfalls mit ein bis drei Fluoratomen substituiert ist, (C₁-C₄)Alkoxy ausgewählt ist;
oder ein pharmazeutisch annehmbares Salz.

7. Verwendung nach Anspruch 6, wobei das SRI-Antidepressivum oder das pharmazeutisch annehmbare Salz davon in dem Medikament in einer Menge von 0,05 mg bis 1500 mg vorhanden ist und das atypische Antipsychotikum oder pharmazeutisch annehmbare Salz davon dem Patienten in einer Menge von 0,05 mg pro Tag bis 1500 mg pro Tag verabreicht wird.

8. Verwendung nach Anspruch 4 oder 6, wobei das atypische Antipsychotikum oder das pharmazeutisch annehmbare Salz davon ausgewählt ist aus:
Abaperidon
7-[3-[4-(6-Fluor-1,2-benzisoxazol-3-yl)-1-piperidinyl]propoxy]-3-(hydroxymethyl)-4H-1-benzopyran-4-on;
Belaperidon
(1a,5a,6a)-3-[2-[6-(4-Fluorphenyl)-3-azabicyclo[3.2.0]-hept-3-yl]ethyl]-2,4(1H,3H)chinazolindion;
Clozapin
8-Chlor-11-(4-methyl-1-piperazinyl)-5H-dibenzo[b,e][1,4]diazepin;
Iloperidon
1-[4-[3-[4-(6-Fluor-1,2-benzisoxazol-3-yl)-1-piperdinyl]-3-methoxyphenyl]ethanon;
Olanzapin
2-Methyl-4-(4-methyl-1-piperazinyl)-10H-thieno[2,3-b][1,5]-benzodiazepin;
Perospiron
Cis-2-[4-[4-(1,2-benzisothiazol-3-yl)-1-piperazinyl]butyl]-hexahydro-1H-isoindol-1,3(2H)-dion;
Risperidon
3-[2-[4-(6-Fluor-1,2-benzisoxazol-3-yl)piperidin]ethyl]-2-methyl-6,7,8,9-tetrahydro-4H-pyrido[1,2-α]pyrimidin-4-on;
Sertindol
1-[2-[4-[5-Chlor-1-(4-fluorphenyl-1H-indol-3yl]-1-piperidinyl]ethyl]imidazolidin-2-on;
Tiospiron
8-[4-[4-(1,2-Benzisothiazol-3-yl)-1-piperazinyl]butyl]-8-azaspiro[4.5]decan-7,9-dion;
Ziprasidon
5-[2-[4-(1,2-Benzisothiazol-3-yl)-1-piperazinyl]ethyl]-6-chlor-1,3-dihydro-2H-indol-2-on;
Zotepin
2-[(8-Chlordibenzo[b,f]thiepin-10-yl)oxy]-N,N-dimethyl-ethanamin;
Quetiapin
5-[2-(4-Dibenzo[b,f][1,4]thiazepin-11-yl-1piperazinyl)ethoxy]ethanol; und
Blonanserin
2-(4-Ethyl-1-piperazinyl)-4-(4-fluorphenyl)-5,6,7,8,9,10-hexahydrocycloocta[b]pyridin.

## Revendications

1. Composition pharmaceutique pour le traitement de la dépression, du syndrome obsessionnel compulsif et de la psychose chez un mammifère, comprenant : (a) un composé qui présente une activité, respectivement, comme antidépresseur SRI, ou un de ses sels pharmaceutiquement acceptables ; (b) un agent antipsychotique atypique ou un de ses sels pharmaceutiquement acceptables ; et (c) un support pharmaceutiquement acceptable ; dans laquelle les agents actifs "a" et "b" ci-dessus sont présents en des quantités qui rendent la composition efficace dans le traitement, respectivement, de l'anxiété ou de la dépression, du syndrome obsessionnel compulsif et de la psychose, avec une efficacité améliorée, et dans laquelle dans l'agent antidépresseur SRI ou son sel pharmaceutiquement acceptable est choisi parmi des composés de formule II, définie ci-dessous, et leurs sels pharmaceutiquement acceptables : 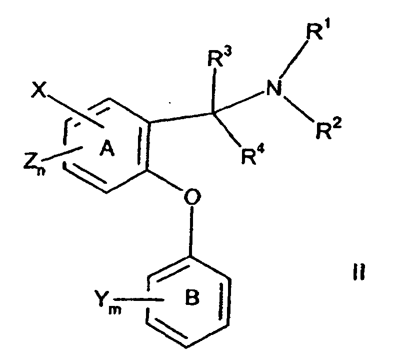 formule dans laquelle le noyau phényle A et le noyau phényle B peuvent chacun, indépendamment, être remplacés par un groupe naphtyle, et dans laquelle, lorsque le noyau phényle A est remplacé par un groupe naphtyle, l'atome d'oxygène d'éther de la formule II et l'atome de carbone auquel R³, R⁴ et NR¹R² sont fixés, sont fixés à des atomes de carbone de noyau adjacents du groupe naphtyle et aucun desdits atomes de carbone de noyau adjacents n'est également adjacent à un atome de carbone de noyau condensé dudit groupe naphtyle ;
n et m sont choisis, indépendamment, entre 1, 2 et 3 ;
R¹ et R² sont choisis, indépendamment, entre un atome d'hydrogène, des groupes alkyle en C₁ à C₄, alcényle en C₂ à C₄ et alcynyle en C₂ à C₄, ou bien R¹ et R², conjointement avec l'atome d'azote auquel ils sont fixés, forment un noyau saturé tétra- à octogonal contenant un ou deux hétéroatomes, y compris l'atome d'azote auquel R¹ et R² sont fixés, dans lequel le second hétéroatome, lorsqu'il est présent, est choisi entre l'oxygène, l'azote et le soufre, et ledit noyau peut être facultativement substitué à des sites de liaisons disponibles avec un à trois substituants choisis, indépendamment, entre des substituants hydroxy et alkyle en C₁ à C₆ ;
R³ et R⁴ sont choisis, indépendamment, entre un atome d'hydrogène et un groupe alkyle en C₁ à C₄ facultativement substitué avec 1 à 3 atomes de fluor, ou bien R³ et R⁴, conjointement avec l'atome de carbone auquel ils sont fixés, forment un noyau carbocyclique saturé tétra- à octogonal, et ledit noyau peut être facultativement substitué à des sites de liaisons disponibles avec un à trois substituants choisis, indépendamment, entre des substituants hydroxy et alkyle en C₁ à C₆ ;
ou bien R² et R³, conjointement avec l'atome d'azote auquel R² est fixé, l'atome de carbone auquel R³ est fixé, forment un noyau saturé tétra- à octogonal contenant un ou deux hétéroatomes, y compris l'atome d'azote auquel R² est fixé, dans lequel le second hétéroatome, lorsqu'il est présent, est choisi entre l'oxygène, l'azote et le soufre, et ledit noyau peut être facultativement substitué au niveau de sites de liaisons disponibles avec un à trois substituants choisis, indépendamment, entre des substituants hydroxy et alkyle en C₁ à C₆ ;
chaque groupe X est choisi, indépendamment, entre des groupes phényle, hétéroaryle et hétérocycliques, et chaque groupe X peut être en outre substitué avec un atome d'hydrogène, un groupe halogéno, alkyle en C₁ à C₄ facultativement substitué avec un à trois atomes de fluor, alkoxy en C₁ à C₄ facultativement substitué avec un à trois atomes de fluor, cyano, nitro, amino, hydroxy, carbonyle, alkylamino en C₁ à C₄, di(alkyle en C₁ à C₄)amino, NR⁵(C=O)(alkyle en C₁ à C₄), SO₂NR⁵R⁶ ou SOₚ(alkyle en C₁ à C₆), dans lequel R⁵ et R⁶ sont choisis, indépendamment, entre un atome d'hydrogène et un groupe alkyle en C₁ à C₆, et p est égal à zéro, un ou deux ;
chaque groupe Y est choisi, indépendamment, entre un atome d'hydrogène, des groupes halogéno, alkyle en C₁ à C₄ facultativement substitué avec un à trois atomes de fluor, alkoxy en C₁ à C₄ facultativement substitué avec un à trois atomes de fluor, cyano, nitro, amino, alkylamino en C₁ à C₄, di (alkyle en C₁ à C₄)amino, NR⁵(C=O) (alkyle en C₁ à C₄), SO₂NR⁵R⁶ ou SOₚ(alkyle en C₁ à C₆), dans lequel R⁵ et R⁶ sont choisis, indépendamment, entre un atome d'hydrogène et un groupe alkyle en C₁ à C₆, et p est égal à zéro, un ou deux ; et
chaque groupe Z est choisi, indépendamment, entre un atome d'hydrogène, des groupes halogéno, alkyle en C₁ à C₄ facultativement substitué avec un à trois atomes de fluor, et alkoxy en C₁ à C₄ ;
ou un de ses sels pharmaceutiquement acceptables.

2. Composition pharmaceutique suivant la revendication 1, dans laquelle l'agent antipsychotique atypique ou un de ses sels pharmaceutiquement acceptables est choisi entre :
7-[3-[4-(6-fluoro-1,2-benzisoxazol-3-yl)-1-pipéridinyl]-propoxy]-3-(hydroxyméthyl)-4H-1-benzopyran-4-one ;
bélapéridone
(1a,5a,6a)-3-[2-[6-(4-fluorophényl)-3-azabicyclo[3.2.0]-hept-3-yl]éthyl]-2,4(1H,3H)quinazolinedione ;
clozapine
8-chloro-11-(4-méthyl-1-pipérazinyl)-5H-dibenzo[b,e][1,4]-diazépine
ilopéridone
1-[4-[3-[4-(6-fluoro-1,2-benzisoxazol-3-yl)-1-pipéridinyl]-3-méthoxyphényl]éthanone ;
olanzapine
2-méthyl-4-(4-méthyl-1-pipérazinyl)-10H-thiéno[2,3-b][1,5]-benzodiazépine ;
pérospirone
cis-2-[4-[4-(1,2-benzisothiazol-3-yl)-1-pipérazinyl]butyl]-hexahydro-1H-isoindole-1,3(2H)-dione ;
rispéridone
3-[2-[4-(6-fluoro-1,2-benzisoxazol-3-yl)pipéridino]éthyl]-2-méthyl-6,7,8,9-tétrahydro-4H-pyrido[1,2-α]pyridimin-4-one ;
serlindole
1-[2-[4-[5-chloro-1-(4-fluorophényl-1H-indol-3yl]-1-pipéridinyl]éthyl]imidazolidin-2-one ;
tiospirone
8-[4-[4-(1,2-benzisothiazol-3-yl)-1-pipérazinyl]butyl]-8-azaspiro[4,5]décane-7,9-dione ;
ziprasidone
5-[2-[4-(1,2-benzisothiazole-3-yl)-1-pipérazinyl]éthyl]-6-chloro-1,3-dihydro-2H-indol-2-one ;
zotépine
2-[(8-chlorodibenzo[b,f]thiépin-10-yl)oxy]-N,N-diméthyléthanamine ;
quétiapine
5-[2-(4-dibenzo[b,f][1,4]thiazépin-11-yl-1pipérazinyl)-éthoxy]éthanol ; et
blonanserine
2-(4-éthyl-1-pipérazinyl)-4-fluorophényl)-5,6,7,8,9,10-hexahydro-cycloocta[b]pyridine.

3. Composition pharmaceutique suivant la revendication 1, dans laquelle l'antidépresseur SRI, ou son sel pharmaceutiquement acceptable, est présent en une quantité de 0,05 mg à 1500 mg et l'agent antipsychotique atypique, ou son sel pharmaceutiquement acceptable, est présent en une quantité de 0,5 mg à 1500 mg.

4. Utilisation d'une composition pharmaceutique suivant l'une quelconque des revendications 1 à 3, dans la production d'un médicament destiné au traitement de l'anxiété ou de la dépression, du syndrome obsessionnel compulsif et de la psychose.

5. Utilisation suivant la revendication 4, dans laquelle l'antidépresseur SRI, ou son sel pharmaceutiquement acceptable, est présent dans ledit médicament en une quantité de 0,05 mg à 1500 mg, et l'agent antipsychotique atypique, ou son sel pharmaceutiquement acceptable, est présent en une quantité de 0,05 mg à 1500 mg.

6. Utilisation d'un antidépresseur SRI, ou d'un de ses sels pharmaceutiquement acceptables, dans la production d'un médicament destiné au traitement de l'anxiété ou de la dépression, du syndrome obsessionnel compulsif et de la psychose, pour l'administration simultanément, séparément ou séquentiellement à des patients recevant un agent antipsychotique atypique ou un de ses sels pharmaceutiquement acceptables, dans laquelle l'anti-dépresseur SRI ou son sel pharmaceutiquement acceptable est choisi parmi des composés de formule II 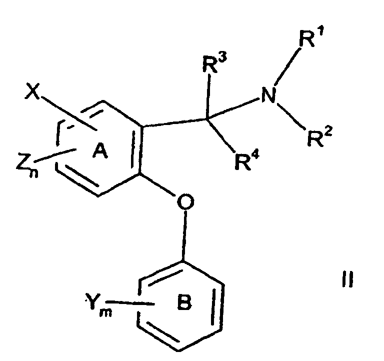 dans laquelle le noyau phényle A et le noyau phényle B peuvent être remplacés chacun, indépendamment, par un groupe naphtyle, et dans laquelle, lorsque le noyau phényle A est remplacé par un groupe naphtyle, l'atome d'oxygène d'éther de la structure I et l'atome de carbone auquel R³, R⁴ et NR¹R² sont fixés, sont fixés à des atomes de carbone de noyau adjacents du groupe naphtyle et aucun desdits atomes de carbone de noyau adjacents n'est également adjacent à un atome de carbone de noyau condensé dudit groupe naphtyle ;
n et m sont choisis, indépendamment, entre 1, 2 et 3 ;
R¹ et R² sont choisis, indépendamment, entre un atome d'hydrogène, des groupes alkyle en C₁ à C₄, alcényle en C₂ à C₄ et alcynyle en C₂ à C₄, ou bien R¹ et R², conjointement avec l'atome d'azote auquel ils sont fixés, forment un noyau saturé tétra- à octogonal contenant 1 ou 2 hétéroatomes, y compris l'atome d'azote auquel R¹ et R² sont fixés, dans lequel le second hétéroatome, lorsqu'il est présent, est choisi entre l'oxygène, l'azote et le soufre, et dans lequel ledit noyau peut être facultativement substitué à des sites de liaisons disponibles avec un à trois substituants choisis, indépendamment, entre des substituants hydroxy et alkyle en C₁ à C₆ ;
R³ et R⁴ sont choisis, indépendamment, entre un atome d'hydrogène et un groupe alkyle en C₁ à C₄ facultativement substitué avec un à trois atomes de fluor, ou bien R³ et R⁴, conjointement avec l'atome de carbone auquel ils sont fixés, forment un noyau carbocyclique saturé tétra- à octogonal, et ledit noyau peut être facultativement substitué à des sites de liaisons disponibles avec un à trois substituants choisis, indépendamment, entre des substituants hydroxy et alkyle en C₁ à C₆ ;
ou bien R² et R³, conjointement avec l'atome d'azote auquel R² est fixé, et l'atome de carbone auquel R³ est fixé, forment un noyau saturé tétra- à octogonal contenant un ou deux hétéoratomes, y compris l'atome d'azote auquel R² est fixé, dans lequel le second hétéroatome, lorsqu'il est présent, est choisi entre l'oxygène, l'azote et le soufre, et ledit noyau peut être facultativement substitué à des sites de liaisons disponibles avec un à trois substituants choisis, indépendamment, entre des substituants hydroxy et alkyle en C₁ à C₆ ;
chaque groupe X est choisi, indépendamment, entre des groupes phényle, hétéroaryle et hétérocycliques, et chaque groupe X peut être en outre substitué avec un atome d'hydrogène, des groupes halogéno, alkyle en C₁ à C₄ facultativement substitué avec un à trois atomes de fluor, alkoxy en C₁ à C₄ facultativement substitué avec un à trois atomes de fluor, cyano, nitro, amino, hydroxy, carbonyle, alkylamino en C₁ à C₄, di(alkyle en C₁ à C₄)amino, NR⁵(C=O)(alkyle en C₁ à C₄), SO₂NR⁵R⁶ ou SOₚ(alkyle en C₁ à C₆), dans lequel R⁵ et R⁶ sont choisis, indépendamment, entre un atome d'hydrogène et un groupe alkyle en C₁ à C₆, et p est égal à zéro, un ou 2 ;
chaque groupe Y est choisi, indépendamment, entre un atome d'hydrogène, des groupes halogéno, alkyle en C₁ à C₄ facultativement substitué avec un à trois atomes de fluor, alkoxy en C₁ à C₄ facultativement substitué avec un à trois atomes de fluor, cyano, nitro, amino, alkylamino en Ci à C₄, di(alkyle en C₁ à C₄)amino, NR⁵(C=O)(alkyle en C₁ à C₄), SO₂NR⁵R⁶ et SOₚ(alkyle en C₁ à C₆), dans lesquels R⁵ et R⁶ sont choisis, indépendamment, entre un atome d'hydrogène et un groupe alkyle en C₁ à C₆, et p est égal à zéro, un ou 2 ; et
chaque groupe Z est choisi, indépendamment, entre un atome d'hydrogène, des groupes halogéno, alkyle en C₁ à C₄ facultativement substitué avec un à trois atomes de fluor, et alkoxy en C₁ à C₄ ;
et leurs sels pharmaceutiquement acceptables.

7. Utilisation suivant la revendication 6, dans laquelle l'antidépresseur SRI, ou un de ses sels pharmaceutiquement acceptables, est présent dans ledit médicament en une quantité de 0,05 mg à 1500 mg, et l'agent antipsychotique atypique, ou son sel pharmaceutiquement acceptable, est administré au patient en une quantité de 0,05 mg par jour à 1500 mg par jour.

8. Utilisation suivant la revendication 4 ou 6, dans laquelle l'agent antipsychotique atypique ou son sel pharmaceutiquement acceptable est choisi entre :
7-[3-[4-(6-fluoro-1,2-benzisoxazol-3-yl)-1-pipéridinyl]-propoxy]-3-(hydroxyméthyl)-4H-1-benzopyran-4-one ;
bélapéridone
(1a,5a,6a)-3-[2-[6-(4-fluorophényl)-3-azabicyclo[3.2.0]-hept-3-yl]éthyl]-2,9(1H,3H)quinazolinedione ;
clozapine
8-chloro-11-(4-méthyl-1-pipérazinyl)-5H-dibenzo[b,e][1,4]-diazépine ;
ilopéridone
1-[4-[3-[4-(6-fluoro-1,2-benzisoxazol-3-yl)-1-pipéridinyl]-3-méthoxyphényl]éthanone ;
olanzapine
2-méthyl-4-(4-méthyl-1-pipérazinyl)-10H-thiéno[2,3-b][1,5]-benzodiazépine ;
pérospirone
cis-2-[4-[4-(1,2-benzisothiazol-3-yl)-1-pipérazinyl]butyl]-hexahydro-1H-isoindole-1,3(2H)-dione ;
rispéridone
3-[2-[4-(6-fluoro-1,2-benzisoxazol-3-yl)pipéridino]éthyl]-2-méthyl-6,7,8,9-tétrahydro-4H-pyrido[1,2-α]pyridimin-4-one ;
serlindole
1-[2-[4-[5-chloro-1-(4-fluorophényl-1H-indol-3yl]-1-pipéridinyl]éthyl]imidazolidin-2-one ;
tiospirone
8-[4-[4-(1,2-benzisothiazol-3-yl)-1-pipérazinyl]butyl]-8-azaspiro[4,5]décane-7,9-dione ;
ziprasidone
5-[2-[4-(1,2-benzisothiazole-3-yl)-1-pipérazinyl]éthyl]-6-chloro-1m3-dihydro-2H-indol-2-one ;
zotépine
2-[(8-chlorodibenzo[b,f]thiépin-10-yl)oxy]-N,N-diméthyléthanamine ;
quétiapine
5-[2-(4-dibenzo[b,f][1,4]thiazépin-17-yl-lpipéraz-inyl)-éthoxy]éthanol ; et
blonanserine
2-(4-éthyl-1-pipérazinyl)-4-fluorophényl)-5,6,7,8,9,10-hexahydro-cycloocta[b]pyridine.
